# EUROPEAN PATENT APPLICATION

(11) **EP 2 886 144 A1**
(43) Date of publication of application: **24.06.2015**
(21) Application number: 13199015.2
(22) Date of filing: 20.12.2013
(51) Int. Cl.: A61M 5/20, A61M 5/32

(54) **Drug delivery device**

(71) Applicant: Sanofi-Aventis Deutschland GmbH, 65929 Frankfurt am Main (DE)
(72) Inventor: The designation of the inventor has not yet been filed

(57) **Abstract**

The invention refers to a drug delivery device (2) comprising a housing (2.1) capable of receiving and containing a container (syringe 3) and a needle safety device (1). The needle safety device (1) comprises a needle cap (5.1) arranged to cover and protect a needle (4), a needle shield (6) relatively movable with respect to the housing (2.1) to cover and protect the needle (4) and a needle cap remover (7). The needle cap remover (7) has an outer part (8) adapted to be arranged to the housing (2.1) and to cover the needle shield (6) and partly the housing (2.1) and an inner part (9) adapted to be arranged to the needle cap (5.1).

## Description

### Technical Field

The invention relates to a drug delivery device comprising such a needle safety device.

### Background of the Invention

Many drug delivery devices of the prior art, such as auto-injectors, syringes, have been developed for self-administration of the drug.

To protect the needle of the drug delivery device from damage or to protect people from needle-prick injuries before using of the device, the needle of the drug delivery device is covered by a protective needle cap of a flexible material which can be encased by a rigid, in particular a plastic needle shield, the so-called rigid needle shield (shortly named RNS).

In order to prepare the drug delivery device for delivering a dose the protective needle cap has to be removed from the needle. This may be done by gripping the protective needle cap and pulling it away from the needle. This will usually result in an exposed needle which is undesirable in terms of needle safety or for person with a needle phobia. In order to solve that problem the needle of the drug delivery device could be covered by a needle shield or shroud in a manner to hide the needle when the protective needle cap is removed.

### Summary of the Invention

It is an object of the present invention to provide a drug delivery device that reduces the risk of needle-prick injuries and needle damages in particular before using the device and which is simple to use, manufacture and assembling.

The object is achieved by a drug delivery device according to claim 1.

Preferred embodiments of the invention are given in the dependent claims.

According to the invention, a drug delivery device comprises a housing capable of receiving and containing a container and a needle safety device which comprises a needle cap or a rigid needle shield encasing the needle cap arranged to cover and protect a needle, a needle shield relatively movable with respect to the housing to cover and protect the needle and a needle cap remover. The needle cap remover has an outer part adapted to be arranged to the housing and to cover the needle shield and partly the housing and an inner part adapted to be arranged to the needle cap or to the rigid needle shield encasing the needle cap.

In the context of this specification, the term "back" or "proximal" end of a component or of a device refers to the end closest to the user's hand or furthest away from the delivery or injection site and the term "front" or "distal" end of a component or device refers to the end furthest from the user's hand or closest to the delivery or injection site.

In a possible embodiment, the needle cap remover, in particular the outer part and the inner part are formed as a one part remover. Alternatively, the needle cap remover is formed as a multiple-part remover with a separate inner part and a separate outer part which are loosely coupled to each other, in particular which are relatively movable to each other in a given distance to compensate length tolerances.

The arrangement and the respective size of the needle cap remover to cover the needle shield fully and the housing of the drug delivery device partly so that the outer part of the needle cap remover forms a cap which slips over the needle shield and the front or distal end of the housing prevents an unintended, in particular a too early movement of the needle shield. Furthermore, the integrated design of the needle cap remover with an inner part which forms the needle cap remover and with an outer part which forms an outer gripping cap provides a rigid design with optimized connections with the needle cap and safe covering of the needle. In particular, only an axial force exerts to the needle cap, thus ensures that the needle will be contaminate-free and thus keep sterile. In a possible embodiment, the needle cap remover has at least one overlapping portion between the needle cap remover and the housing and/or the needle cap remover and the needle cap. The at least one overlapping portion provides a simple compensation of occurred length tolerances of the components of the drug delivery device, in particular of the needle safety device. In particular, occurring length tolerances of the device components are compensated by axial relocatability between the needle cap and the needle cap remover.

According to an embodiment of the invention, a second overlapping portion is provided between the outer part of the needle cap remover and the housing to compensate axial clearance between the needle cap remover and the housing. In particular, in this second overlapping portion occurring length tolerances of the respective components can be compensated by a given maximal axial relocatability between the needle cap remover and the housing.

According to an additional feature of the invention, an inner surface of the outer part and an outer surface of the housing in the second overlapping portion are correspondingly formed. In particular, the inner surface and the outer surface are profiled e.g. with grooves, notches, recesses, ridges and/or ribs to provide an adjustable snap-fit-connection and/or positive locking connection and/or form-fitting connection.

Additionally or alternatively, a first overlapping portion is provided between the inner part of the needle cap remover and the outer part of the needle cap remover to compensate axial clearance between the needle cap remover and the needle cap or the rigid needle shield encasing the needle cap. In particular, in this first overlapping portion occurring length tolerances of the respective components can be compensated by a given maximal axial relocatability between the separate inner part and the outer part of the needle cap remover, and thus between the needle cap remover and the needle cap or the rigid needle shield encasing the needle cap.

In other words: The multiple-part design of the needle cap remover comprising the inner part and the outer part which are coupled to each other by corresponding projections allows a limited setting range of the needle cap remover with respect to the needle cap. In a possible embodiment, the outer part of the needle cap remover is designed as an outer cap which covers and protects the inner needle shield and thus prevents an unintended, in particular a too early movement of the needle shield.

The outer part comprises a front end having an opening which at its edge exhibits a projection directed inwards adapted to catch a front projection of the inner part so as to couple the outer part and the inner part within the given first overlapping portion. The inner part comprises a back end having a grip member adapted to grip a back end of the needle cap so as to couple the needle cap remover and the needle cap. In a possible embodiment, the grip member is formed as locking hooks.

In an exemplary embodiment, the projection of the outer part is formed as at least two opposite arms which ends are angled. In particular, the ends of the opposite arms could be angled away from each other or otherwise to engage the corresponding front projection of the inner part. Depending on the material of the inner and outer part of the needle cap remover, at least one of the parts, in particular the opposite arms or the front projection are flexible, e.g. are made of a flexible material or soft plastic. Due to a flexible design of the angled ends, the flexible arms of the outer part could be linked to the inner part in such a manner that they are pivoted or swiveled to approach each other so that they are carried over the rigid front projection of the inner part when the outer part is assembled onto or into the inner part. Otherwise, the flexible front projection may be pivoted or swiveled away from each other by the rigid arms of the outer part when the outer part is assembled to the inner part, e.g. onto or into the inner part. After assembling of the outer and inner parts, the front projection of the inner part and the projection of the outer part are correspondingly inclined to each other in such a manner that they are coupled to each other to allow a common axial movement after assembling.

In a possible embodiment, the front projection of the inner part is correspondingly formed to the projection of the outer part. In particular the front projection of the inner part is formed as at least two protruding tabs. The protruding tabs are protruded from the inner surface of the inner part and are directed inwards in an angle to be gripped by the also angled ends of the arms of the outer part. During assembling of the outer part onto or into the inner part, the angled ends of the arms and the angled protruding tabs of the front projection are coupled and engaged by a positive-locking connection and/or form-fitting connection, e.g. snap-fit-connection or latching connection.

In a further embodiment, the front projection of the inner part is formed as flexible legs or a stem with flexible legs which protrude from the front end of the inner part and directed inwards through the opening of the outer part.

According to another embodiment of the invention, the grip member of the inner part is formed as a back projection of the inner part. The back projection bents into the inner part to engage the back end of the needle cap by a positive-locking connection and/or form-fitting connection. In particular, the back projection is formed as at least two legs directed inwards to engage the back end of the needle cap or the rigid needle shield encasing the needle cap.

According to an alternative embodiment, the grip member of the inner part of the needle cap remover may be formed with a profiled surface adapted to grip lateral surfaces of the needle cap or the rigid needle shield encasing the needle cap e.g. by friction connection and/or positive-locking connection and/or form-fitting connection. In particular, the inner surface of the inner part is roughened or lightly ribbed or grooved or coated by a soft coating material.

Alternatively or additionally, the needle cap or the encasing rigid needle shield has an outer surface comprising grip elements adapted to be gripped by the grip member of the inner part of the needle cap remover e.g. by positive-locking connection and/or form-fitting connection and/or friction connection. In particular, the grip elements of the needle cap or encasing the rigid needle shield may be formed as a profiled outer surface, e.g. as ribs, hooks, domes, grooves and/or protruding pegs, arms. The coupling between the inner part of the needle cap remover and the needle cap or the rigid needle shield encasing the needle cap is designed in such a manner that after assembling during an axial movement of the needle cap remover the needle cap or the rigid needle shield encasing the needle cap is removed from the needle together with the needle cap remover.

In yet a further embodiment, the inner part of the needle cap remover is inserted into an opening of the needle shield and is also inserted in this opening between the needle cap or the rigid needle shield encasing the needle cap and a spring which engages on one end at the housing and on the other end at the needle shield. The opening or the hole in the needle shield has an appropriate width to allow the insertion of the inner part of the needle cap remover.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given herein below and the accompanying drawings which are given by way of illustration only, and thus, are not limitive of the present invention, and wherein:
- Figure 1: shows a partial longitudinal cross section of an exemplary embodiment of a front part of a drug delivery device with a needle safety device in a protection position,
- Figure 2: shows a partial longitudinal cross section of an exemplary embodiment of a front part of a drug delivery device with a needle safety device in an intermediate position during removing of a needle cap,
- Figure 3: shows a perspective view of an exemplary embodiment of a needle cap remover,
- Figure 4: shows an enlarged view of the connection between a needle cap remover and a needle cap,
- Figure 5: shows a perspective view of another exemplary embodiment of a needle safety device,
- Figure 6: shows an enlarged view of another exemplary connection between a needle cap remover and a needle cap,
- Figures 7 and 8: show partial longitudinal cross sections of exemplary embodiments of a front part of a drug delivery device with a needle safety device in a protection position.
- Figures 9 and 10: show an explosion view of a needle safety device of a drug delivery device
- Figure 11: shows a partial longitudinal section of an embodiment of an alternative inner part of a needle cap remover,
- Figure 12: shows the inner part of the needle cap remover according to figure 9 assembled to a needle cap of a drug delivery device in a longitudinal section,
- Figure 13: shows a perspective view of an embodiment of a clamp-like inner part of a needle cap remover, and
- Figures 14 and 15: show alternative embodiments of the inner part of the needle cap remover.

Corresponding parts are marked with the same reference symbols in all figures.

### Detailed Description of Preferred Embodiments

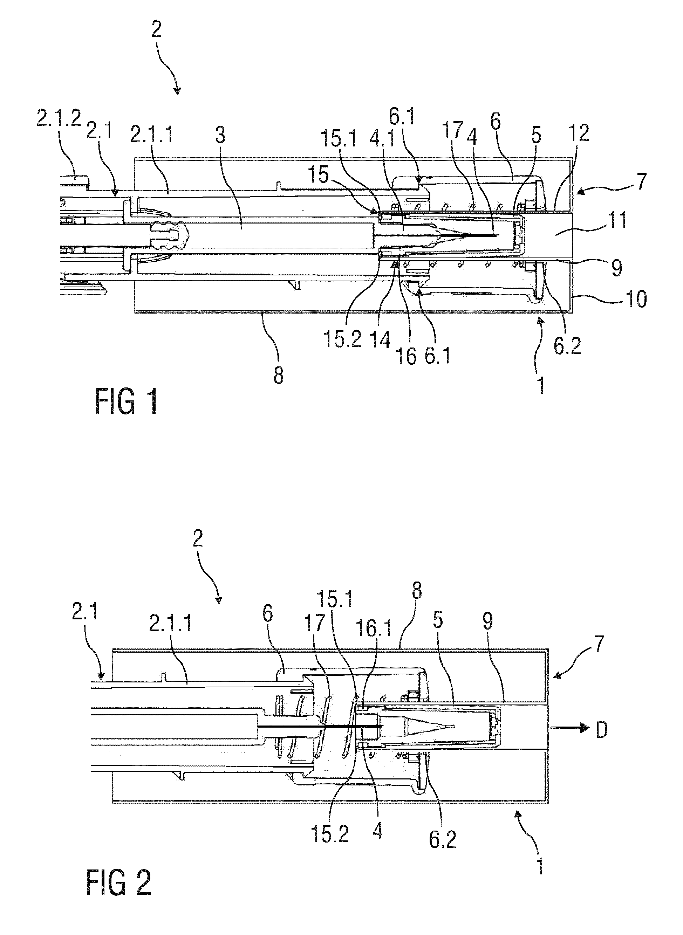

Figure 1 shows one embodiment of the present invention in a partial longitudinal cross section of a needle safety device 1 for a drug delivery device 2.

The drug delivery device 2 is a pre-filled syringe 3 having a needle 4. The needle 4 may be fixed to the distal end of the syringe 3 or removable therefrom, as a matter of design choice. The syringe 3 is hold in a housing 2.1 of the drug delivery device 2. Alternatively, the drug delivery device could be an auto-injector with a container, an injection pen or a syringe to be filled or another medicament container or any other application with comparable functional principle. The housing 2.1 may be designed as a one part or multiple part housing capable of receiving and containing the container, in the embodiment the syringe 3. In the shown embodiment the housing 2.1 comprises a front part 2.1.1 and a back part 2.1.2.

The needle safety device 1 comprises a rigid needle shield 5 encasing a needle cap 5.1, a needle shield 6 and a needle cap remover 7 which are adapted to be connected to each other by friction connection and/or positive locking connection and/or form-fitting connection. As it can be seen, the needle shield 6 is being mounted on the housing 2.1 of the drug delivery device 2 in a movable manner. In particular, the needle shield 6 is movably mounted on the front part 2.1.1 of the housing 2.1.

The needle cap 5.1 encased by the rigid needle shield 5 is being mounted on the needle 4 in a separable manner. The needle cap remover 7 is being mounted on the rigid needle shield 5 in a secured manner and on the housing 2.1, in particular on the front part 2.1.1, in a separable manner.

The rigid needle shield 5 is designed as a protective needle shield so called as a rigid needle shield (shortly named RNS) which covers the needle 4 before use to protect it against damages during transport or travel and to stay it sterile during the assembly process. In the shown embodiment the rigid needle shield 5 encases the needle cap 5.1 which is made of a flexible material e.g. of rubber or silicon.

In an alternative, not shown embodiment, the needle safety device 1 comprises only the needle cap 5.1 without the rigid needle shield so that the needle cap remover 7 is directly arranged onto the needle cap 5.1. The function and the arrangement of the needle cap remover 7 directly onto the needle cap 5.1 or onto the rigid needle shield 5 encasing the needle cap 5.1 are identical and do not differ from each other. Thus, the following description regarding to a needle cap remover arranged onto a rigid needle shield encasing a needle cap also applies to a needle cap remover directly arranged onto the needle cap without an encasing.

The needle cap remover 7 is formed as an integrated unit with an inner part 9 and an outer part 8. The inner part 9 is adapted to be arranged to the rigid needle shield 5 and forms an inner gripper for the rigid needle shield 5. The outer part 8 is adapted to be arranged to the housing 2.1 and forms an outer gripping surface for a user of the device. The invention prevents a misuse or unintended activation and optimizes the connection between the rigid needle shield 5 and the needle cap remover 7.

As not shown in the longitudinal cross sections of the needle safety device 1, the components, in particular the needle cap remover 7 with the inner part 9 and the outer part 8 as well as the needle cap 5.1, have tubular or cylindrical shapes. They may be having different shapes and may have oval or polygonal shapes in cross section.

The needle cap remover 7 has a respective size and is arranged to the drug delivery device 2 and the rigid needle shield 5 in such a manner that it cover the needle shield 6 fully and the housing 2.1 in part, in particular only the front part 2.1.1. The outer part 8 of the needle cap remover 7 forms an outer gripping surface in form of a cap which slips over the needle shield 6 and the front part 2.1.1 of the housing 2.1 during assembling so that an early movement of the needle shield 6 is prevented.

In detail, the needle cap 5.1 is arranged onto a needle hub 4.1 to directly cover and protect the needle 4. The needle cap 5.1 may be connected to a needle hub 4.1 of the needle 4 by means of a friction connection and/or positive locking connection. The needle cap 5.1 is preferably of a resilient material, especially of a soft elastomer, for example rubber or a thermoplastic elastomer (TPE). The rigid needle shield 5 encasing the needle cap 5.1 is preferably of a rigid material and is formed as a protective needle shield, e.g. as a rigid needle shield.

The needle shield 6 covers and protects the needle 4 before, during and after use and in particular also after removing of the rigid needle shield 5 and the encased needle cap 5.1. The needle shield 6 is movably arranged with respect to the housing 2.1 of the drug delivery device 2. In the shown embodiment, the needle shield 6 is provided at the distal or front end of the housing 2.1. In particular, the needle shield 6 is arranged onto the housing 2.1. Alternatively, the needle shield 6 could be arranged into the housing 2.1.

As shown in figure 1, the needle shield 6 is in the advanced or extended position in which the needle 4 is covered. The needle shield 6 is secured in the advanced position by a spring element 17 which bears at one end on the needle shield 6 and at the other end on the housing 2.1. In this advanced position before use, the needle shield 6 is hold by a positive connection and/or a friction connection and/or a form-fitting connection, e.g. a snap-fit connection 6.1. During use, the needle shield 6 is movable from the advanced position to a retracted position wherein the spring element 17 is tensioned (not shown). After use, the needle shield 6 is movable back to the advanced position in which the needle shield 6 is securely fixed with respect to the housing 2.1.

The needle shield 6 comprises an opening 6.2 through which the needle cap remover 7 is inserted. In particular, the inner part 9 of the needle cap remover 7 is inserted in this opening 6.2 between the rigid needle shield 5 and the spring 17. The opening 6.2 or the hole in the needle shield 6 has an appropriate width to allow the insertion of the inner part 9. In respect to this design, the needle cap remover 7 might be assembled to the device before or after the needle shield 6.

The needle cap remover 7 comprises at least the outer part 8 and the inner part 9. The outer part 8 is adapted to be arranged at the housing 2.1 and is formed as an outer gripping surface. Preferably, the outer part 8 is releasably coupled to the housing 2.1 by a friction connection and/or a form-fitting connection. In detail, the outer part 8 of the needle cap remover 7 is designed as an outer gripping cap which covers and protects the inner needle shield 6 and thus prevents an unintended, in particular a too early movement of the needle shield 6.

The inner part 9 is adapted to carry the rigid needle shield 5 by a friction connection and/or a positive locking connection wherein the rigid needle shield 5 forms the so-called protective needle shield or rigid needle shield (shortly named RNS) of the needle safety device 1.

The outer part 8 comprises a front end 10 having an opening 11 which at its edge exhibits a projection 12 directed inwards. The inwardly directed projection 12 forms the inner part 9 of the needle cap remover 7.

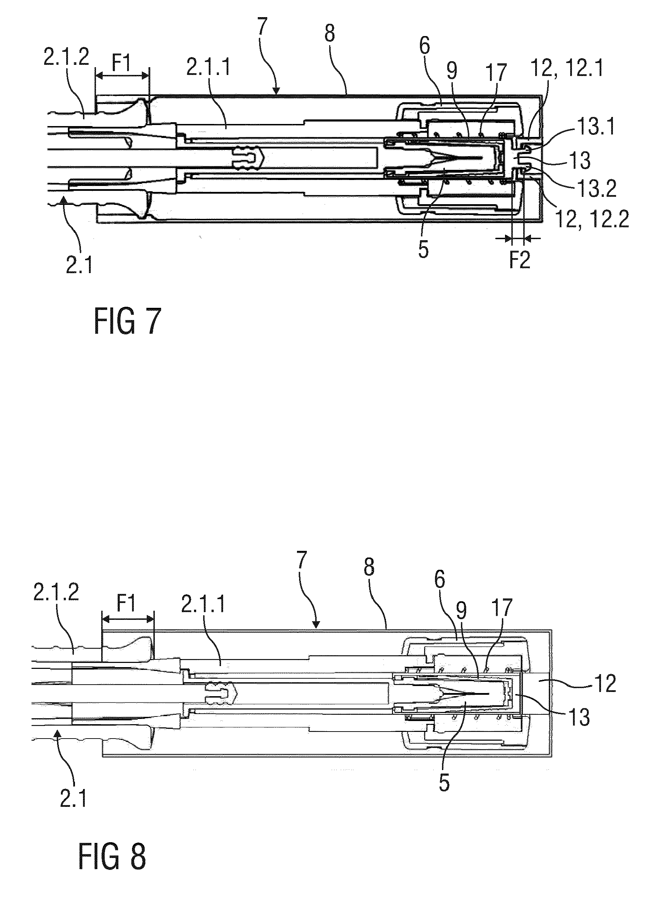

Depending on the design of the needle cap remover 7, the inner part 9 and the outer part 8 may be formed as an integrated unit shown in embodiments according to figures 1 to 6. Alternatively the inner part 9 and the outer part 8 may be formed as separate but securely and non-releasably coupled parts, shown in embodiments according to figures 7 and 8.

Due to the coupling of the outer part 8 with the inner part 9 and the inner part 9 with the rigid needle shield 5, the needle cap 5.1 encased by rigid the needle shield 5 is removed from the needle 4 when the outer part 8 is removed from the drug delivery device 2.

The inner part 9 comprises a back end 14. The back end 14 has a grip member 15 adapted to grip a back end 16 of the rigid needle shield 5 so as to couple the needle cap remover 7 to the rigid needle shield 5.

The grip member 15 is designed as looking hooks 15.1, 15.2 which cooperate with a complementary locking element 16.1, e.g. recess or groove, arranged at the back end 16 of the rigid needle shield 5. The locking hooks 15.1, 15.2 engage the back end 16 of the rigid needle shield 5 by a positive-locking connection and/or form-fitting connection.

Figure 2 shows the drug delivery device 2 with the safety needle device 1 in an intermediate position in which the needle cap remover 7 is slightly removed from the housing 2.1 into direction D. Due to the coupling of the needle cap remover 7 and the rigid needle shield 5 as well as to the encasing and coupling of the needle cap 5.1 by the rigid needle shield 5, the rigid needle shield 5 together with the needle cap 5.1 is correspondingly removed from the needle 4.

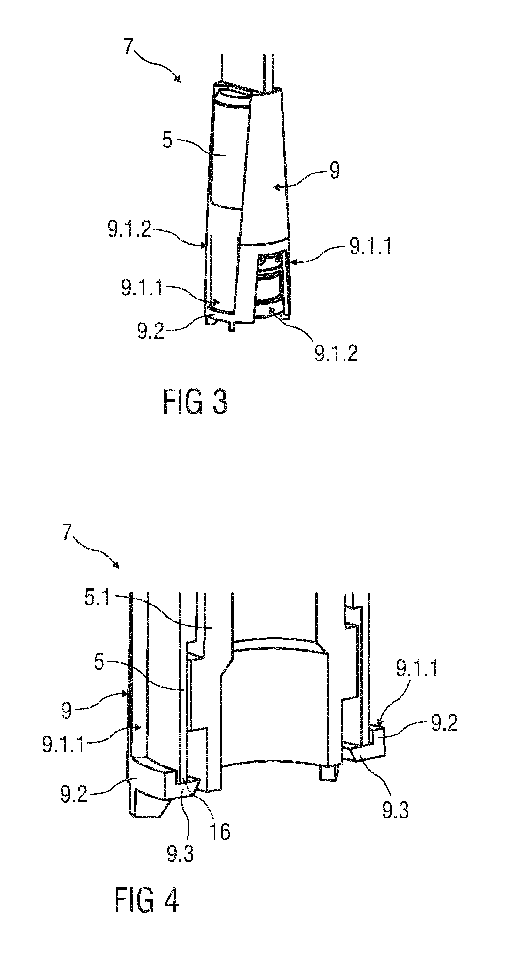

Figure 3 shows a partial perspective view of an exemplary embodiment of a needle cap remover 7, wherein figure 4 shows an enlarged view of the connection between the needle cap remover 7 and the rigid needle shield 5. Figure 3 shows the needle cap 5.1 and inner part 9 of the needle cap remover 7. The needle shield 6 and the outer part 8 are not shown.

The resilient needle cap 5.1 is internally arranged in the rigid needle shield 5 shown in figure 4.

The inner part 9 of the needle cap remover 7 is formed as a cap remover to remove the rigid needle shield 5 together with the encased needle cap 5.1 from the needle 4 before use. To securely couple the needle cap remover 7 to the rigid needle shield 5, the inner part 9 has a tubular form with pairs of elongated windows 9.1.1 and 9.1.2 so that at least the circumferential edges of the windows 9.1.1 are formed as flexible arms. At the back end 9.2 of each window 9.1.1, the respective edges 9.2 equipped with hooks 9.3 engaging the back end 16 of the rigid needle shield 5. When the needle cap remover 7 is inserted into the opening 6.2 of the needle shield 6, the hooks 9.3 at the flexible arms or windows 9.1.1 being movable along the longitudinal axis of the rigid needle shield 5 into the opening 6.2 between the rigid needle shield 5 and the spring element 17 (not shown in figure 3) so that the windows 9.1.1 and thus the hooks 9.3 are deformed, in particular spread outwards, and after having passed the back end 16 of the rigid needle shield 5 the hooks 9.3 are spread back inwardly and securely engaged the back end 16 in a snap-fit connection or form-fitting connection.

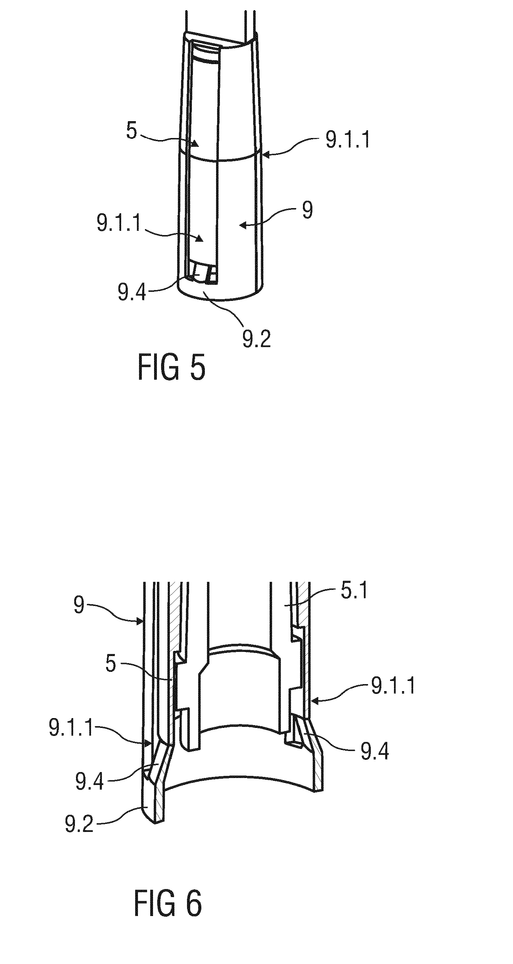

Figures 5 and 6 show a perspective view of another exemplary embodiment of a needle safety device 1 with an enlarged view of another exemplary connection between a needle cap remover 7 and rigid needle shield 5.

The inner part 9 of the needle cap remover 7 has a tubular form with two opposite elongated windows 9.1.1. The inner part 9 is rigid. For an easy assembling of the needle cap remover 7 into the opening 6.2 of the needle shield 6 to grip the rigid needle shield 5, at the back end 9.2 of each window 9.1.1 at least one flexible leg 9.4 extends inwardly into the inner part 9 to engage the back end 16 of the rigid needle shield 5, thereby forming a snap-fit means.

When the needle cap remover 7 is inserted into the opening 6.2 of the needle shield 6, the flexible legs 9.4 being movable along the longitudinal axis of the rigid needle shield 5 into the opening 6.2 between the rigid needle shield 5 and the spring element 17 (not shown in figure 3) so that the legs 9.4 are deformed, in particular spread outwards, and after having passed the back end 16 of the rigid needle shield 5 the legs 9.4 are spread back inwardly and securely engaged the back end 16 in a snap-fit connection, a positive locking connection or form-fitting connection.

Figures 7 and 8 show partial longitudinal cross sections of exemplary embodiments of a front part of a drug delivery device 2 with a needle safety device 1 in a protection position. The difference between the figures 7 and 8 is that in figure 7 the outer body 8 of the needle cap remover 7 is coupled to a front projection 13 of the inner part 9 with a given first overlapping portion F1.

According to figure 8 - a further embodiment of the invention -, the outer part 8 of the needle cap remover 7 and the back part 2.1.2 of the housing 2.1 are overlapped in a given second overlapping portion F2.

Occurring length tolerances of the housing 2.1 and the needle cap remover 7 may be compensated by an axial clearance of an axial relocatability between the needle cap remover 7 and the housing 2.1 within the given first overlapping portion F1 or the given second overlapping portion F2.

According to the concept of compensation of length tolerances in figure 7, the needle cap remover 7 comprises the outer part 8 and the inner part 9 which are non-releasable coupled with the first overlapping portion F1. The inwardly directed projection 12 is adapted to catch a front projection 13 of the inner part 9 so as to non-releasable couple the outer part 8 to the inner part 9 by a positive locking connection and/or a form-fitting connection.

The multiple-part design of the needle cap remover 7 of figure 7 with the separate inner part 9 and the separate outer part 8 which are coupled to each other by corresponding projections 12, 13 allows a limited setting range of the needle cap remover 7 by the first overlapping portion F1. Occurring length tolerances of needle cap 5.1 and the needle cap remover 7 can be compensated by an axial relocatability between the needle cap 5.1 and the needle cap remover 7 within the first overlapping portion F1.

In an exemplary embodiment, the projection 12 of the outer part 8 is designed as at least two opposite arms 12.1, 12.2 which free ends are angled. In particular, the ends of the opposite arms 12.1, 12.2 are angled to each other or alternatively away from each other to engage the corresponding front projection 13 of the inner part 9 e.g. in a clip connection, snap-fit connection, form-fitting connection and/or positive locking connection. Depending on the material of the inner and outer part 8, 9 of the needle cap remover 7, at least one of the parts, in particular the opposite arms 12.1, 12.2 of the outer part 8 are flexible, e.g. are made of a flexible or soft plastic material. Alternatively, the opposite arms 12.1, 12.2 are rigid and the front projection 13 is made of a flexible material.

Due to the flexible design of the arms 12.1, 12.2 with the angled ends, the flexible arms 12.1, 12.2 could be pivoted or swiveled away from each other so that they are carried over the rigid front projection 13 of the inner part 9 during assembling of the outer part 8 to the inner part 9.

Otherwise, the flexible front projection 13 may be pivoted or swiveled so that the hooked ends of the front projection 13 come closer. After assembling of the outer and inner parts 8, 9 the front projection 13 of the inner part 9 and the projection 12 of the outer part 8 are correspondingly inclined to each other in such a manner that they are coupled to each other to allow a common axial movement and in particular an axial relocatability between the rigid needle shield 5 and the needle cap remover 7 within the first overlapping portion F1 and thus within a setting range.

As it is shown in figure 7, the front projection 13 of the inner part 9 is correspondingly formed to the projection 12 of the outer part 8. In particular the front projection 13 is formed as at least two protruding tabs 13.1, 13.2. The protruding tabs 13.1, 13.2 are protruded from the inner surface of the inner part 9 and are directed inwards in an angle to be gripped by the also angled ends of the arms 12.1, 12.2 of the outer part 8.

After assembling of the needle cap remover 7, the angled ends of the arms 12.1, 12.2 and the angled protruding tabs 13.1, 13.2 are coupled and securely engaged, wherein the tabs 13.1, 13.2 define a stop on which the arms 12.1, 12.2 come in abutment in such a manner that when the outer part 8 is gripped and removed from the needle shield 6 the inner part 9 moves too to remove the rigid needle shield 5 and the encased needle cap 5.1 from the needle 4. In particular, the arms 12.1, 12.2 and the protruding tabs 13.1, 13.2 are securely engaged by a positive-locking connection and/or form-fitting connection, e.g. snap-fit-connection or latching connection.

Figure 8 shows that the inner surface of the outer part 8 and the outer surface of the housing 2.1 in the second overlapping portion F2 are correspondingly formed, in particular profiled, e.g. with grooves, ribs, forming press-fit means to provide an adjustable and releasable snap-fit-connection.

The inner part 9 and the outer part 8 are coupled to each other without any compensation of length tolerances. For example the inner part 9 and the outer part 8 are fixed or are formed as an integrated part.

According to the embodiment of figure 8, the needle cap remover 7, in particular the outer part 8 and the inner part 9 are formed as a one part remover.

In summary, depend on the design of the drug delivery device 2 with at least one or two overlapping portions F1, F2 occurring length tolerances may be compensated by axial relocatability of the respective components between the housing 2.1 and the needle cap remover 7 as well as between the needle cap remover 7 and the rigid needle shield 5.

The described different embodiments of a clip-like needle cap remover 7 can be used at smart safety syringe systems. The invention allows that the assembly force to the rigid needle shield 5 (= RNS) is only induced in axial direction. Further, due to the enlarged and cap-like outer part 8 of the needle cap remover 7, which fully cover the needle shield 6, an unintended, in particular a too early movement of the needle shield 6 is prevented.

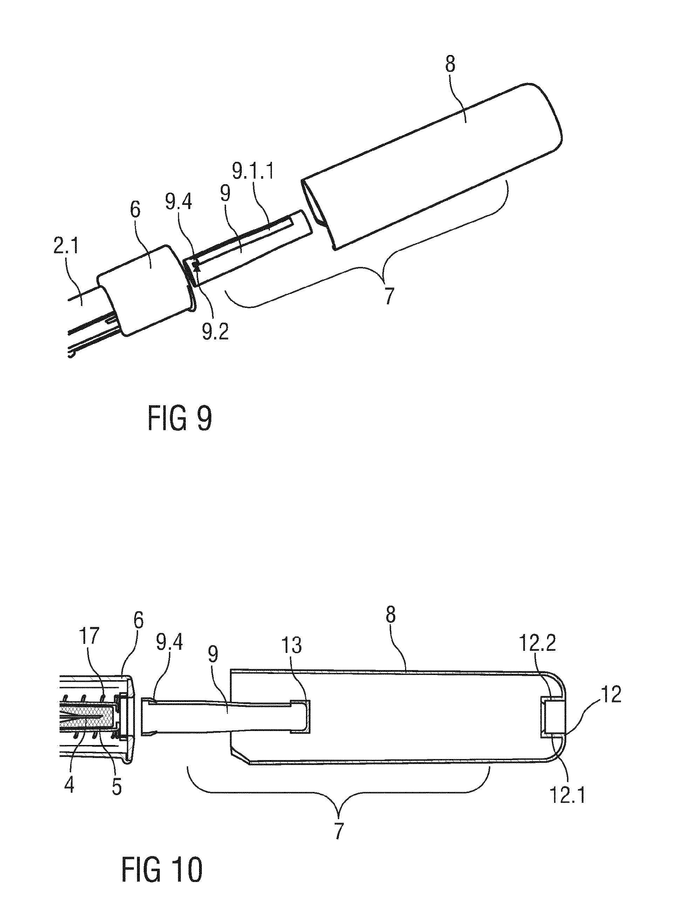

Figures 9 and 10 show an explosion view of an alternative embodiment of a needle safety device 1 comprising an inner part 9 and an outer part 8. The inner part 9 and the outer part 8 comprise corresponding formed projections 12, 13 (shown in figure 10) to couple the inner and outer parts 8, 9 in a snap-fit connection. The projection 12 of the outer part 8 comprises two opposite arms 12.1, 12.2 which engage the corresponding front projection 13 of the inner part 9.

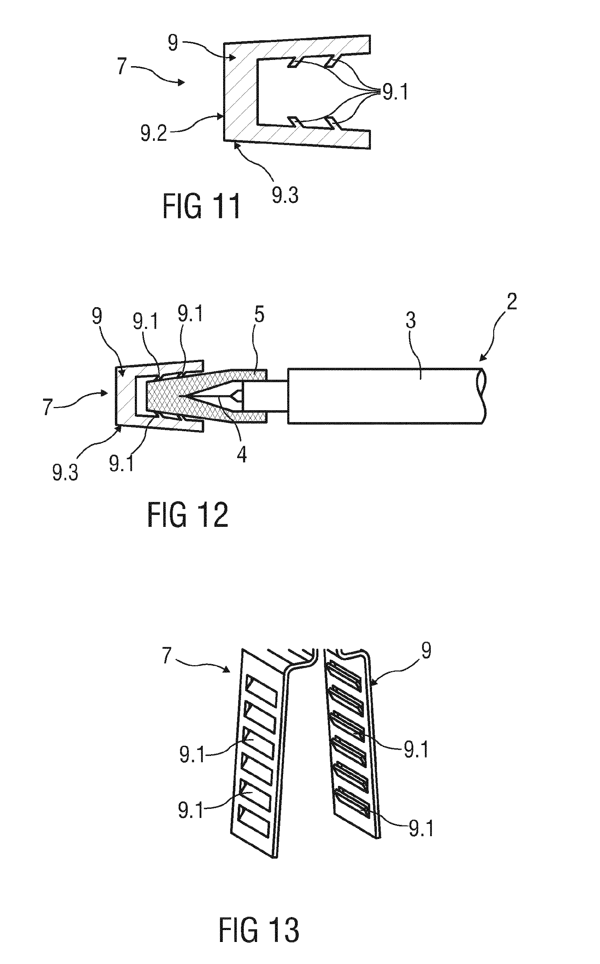

Figure 11 shows a partial longitudinal section of an alternative embodiment of an inner part 9 of a further improved needle cap remover 7. The inner part 9 is formed as a hollow sheet metal part adapted to be arranged onto the rigid needle shield 5. The inner part 9 has a clamp shape or U-shape or the form of a sleeve or cylinder.

The inner part 9 is made of a thin sheet metal. Furthermore, the needle cap remover 7 and the rigid needle shield 5 differs at least in the material and/or the resistance, wherein the rigid needle shield 5 is made of a rigid polypropylene material and the needle cap remover 7 is made of a thin metal sheet profile.

Furthermore, the inner part 9 comprises protrusions 9.1 which extend from the inside wall into the inner hole. Preferably, the inner part 9 comprises a plurality of inwardly directed protrusions 9.1 which are formed as hooks. The hooks may take a number of different forms. Preferably, the hooks are slightly angled and inwardly protrude into the direction of the pull-off-movement of the needle cap remover 7.

Figure 12 shows the inner part 9 formed as a sheet metal part assembled onto a rigid needle shield 5 for a needle 4 of a drug delivery device 2.

During assembling, the inwardly directed edges or protrusions 9.1 slide over the surface of the rigid needle shield 5 when the needle cap remover 7 is pushed upon the rigid needle shield 5. Pulling the needle cap remover 7 into the opposite direction and thus into the removing direction the protrusions 9.1 dig into the outer surface of the rigid needle shield 5 and thus form a positive and/or non-positive connection during removing of the rigid needle shield 5. Thus, the inner part 9 removes the rigid needle shield 5 too by further movement.

Figure 13 shows a perspective view of a possible embodiment of an inner part 9 of a needle cap remover 7. The inner part 9 of the needle cap remover 7 is designed as a clamp-like sheet metal part with a plurality of inwardly directed protrusions 9.1 which are extended from each of the opposite inner surfaces in a row and in a respective distance to each other.

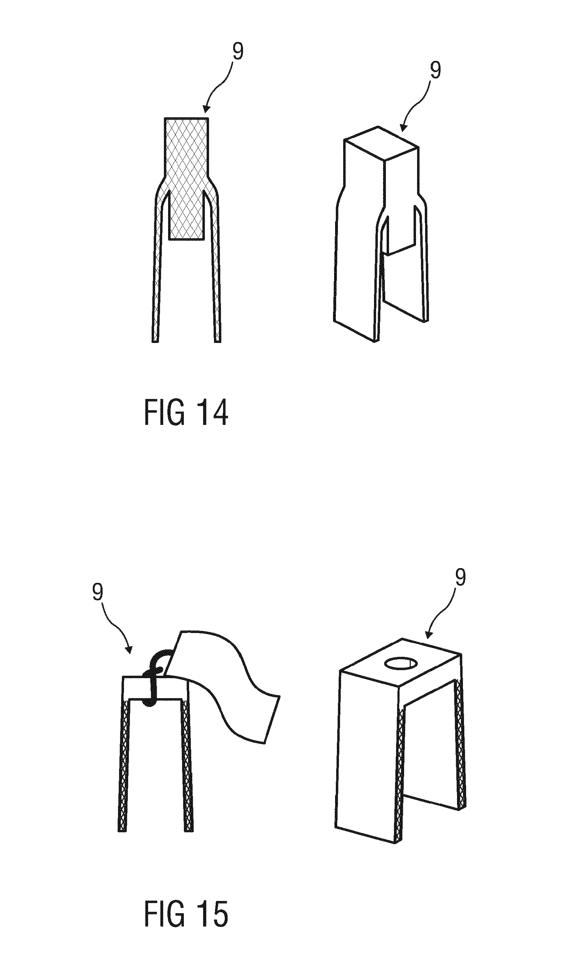

Further alternative embodiments of the inner part of the needle cap remover are shown in figures 14 and 15.

Figure 14 shows an inner part 9 which is formed as a shrinking hose arranged as an outer wall onto the needle cap 5.1 or onto a rigid needle shield 5 encasing the needle cap 5.1 and heat shrunk onto the needle cap 5.1 or onto the encasing rigid needle shield 5.

In an exemplary embodiment, the shrinking hose is formed as a sleeve of high impact resistance, in particular of a thermoplastic material, wherein the coupling between the shrinking hose and the needle cap 5.1 or the encasing rigid needle shield 5 is induced by a heating during assembly. Due to the low wall thickness of the shrinking hose, the shrinking hose is arranged to the needle cap 5.1 or to the encasing rigid needle shield 5 and both together can be assembled to the drug delivery device 2. After the needle cap remover 7 and the needle cap 5.1 or the encasing rigid needle shield 5 are arranged to each other they are heated wherein the shrinking hose shrinks so that the needle cap remover 7 and the needle cap 5.1 or the encasing rigid needle shield 5 are adhesively joined together such that, in use, if the needle cap remover 7 is moved away from the drug delivery device 2, it takes the needle cap 5.1 or the rigid needle shield 5 encasing the inner needle cap 5.1 with it and removes it from the drug delivery device 2 too and thus from the needle 4.

Figure 15 shows an alternative embodiment of an inner part 9 of the needle cap remover 7. The inner part 9 is formed as a fabric tube or textile arranged onto the needle cap 5.1 or onto the rigid needle shield 5 encasing the needle cap 5.1 during assembling and mechanically shrunk onto the needle cap 5.1 or the encasing rigid needle shield 5 during removing. This concept allows an engaging of the needle cap remover 7 to the needle cap 5.1 or the encasing rigid needle shield 5 by a positive and/or non-positive connection, e.g. a form-fitting and/or friction connection. In particular, the needle cap remover 7 and the needle cap 5.1 differs at least in the material and/or the resistance too, wherein the needle cap 5.1 is made of a flexible material, e.g. rubber, and may be encased by the rigid needle shield 5 which is made of a rigid polypropylene cap. The inner part 9 of the needle cap remover 7 is made of a fabric or textile tube of rubber or silicon or similar materials, e.g. rubber-weave hose.

The outer part 8 of the needle cap remover 7 of the alternative embodiments according to figures 9 to 13 may be designed as a long cap as it is described in the embodiments according to figures 1 to 2 and 7 to 8.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
   wherein the group -Lys6-NH2 may be bound to the C-terminus of the Exendin-4 derivative;
   or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
   or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

### List of References

- 1: Needle safety device
- 2: Drug delivery device
- 2.1: housing
- 2.1.1: front part
- 2.1.2: back part
- 3: Syringe
- 4: Needle
- 4.1: Needle hub
- 5: Rigid needle shield
- 5.1: Needle cap
- 6: Needle shield
- 7: Needle cap remover
- 8: Outer part
- 9: Inner part
- 9.1: Protrusion
- 9.1.1: windows
- 9.2: Closed end of the body
- 9.3: Gripping portion
- 9.4: flexible led
- 10: Front end
- 11: Opening
- 12: Projection of the outer part
- 12.1, 12.2: Arms
- 13: Front projection of the inner part
- 13.1, 13.2: Protruding tabs
- 13.3: Dome
- 13.4: Stem
- 13.5: Legs
- 13.5.1: Hooks
- 14: Back end of the inner part
- 15: Grip member of the inner part
- 15.1, 15.2: Legs
- 16: Back end of the needle cap
- 17: spring element

## Claims

1. Drug delivery device (2) comprising:
- a housing (2.1) capable of receiving and containing a container (syringe 3) and
- a needle safety device (1) which comprises
- a needle cap (5.1) or a rigid needle shield (5) encasing the needle cap (5.1) arranged to cover and protect a needle (4);
- a needle shield (6) relatively movable with respect to the housing (2.1) to cover and protect the needle (4);
- a needle cap remover (7) having
- an outer part (8) adapted to be arranged to the housing (2.1) and to cover the needle shield (6) and partly the housing (2.1) and
- an inner part (9) adapted to be arranged to the rigid needle shield (5).

2. Drug delivery device (2) according to claim 1, wherein the needle cap remover (7), in particular the outer part (8) and the inner part (9) are formed as a one part remover or a multiple-part remover.

3. Drug delivery device (2) according to claim 1 or 2, wherein the needle cap remover (7) has at least one overlapping portion (F1, F2) between the needle cap remover (7) and the housing (2.1) and/or the needle cap remover (7) and the rigid needle shield (5).

4. Drug delivery device (2) according to claim 3, wherein a second overlapping portion (F2) is provided between the outer part (8) of the needle cap remover (7) and the housing (2.1).

5. Drug delivery device (2) according to claim 4, wherein an inner surface of the outer part (8) and an outer surface of the housing (2.1) in the second overlapping portion (F2) are correspondingly formed, in particular profiled.

6. Drug delivery device (2) according to any of the preceding claims, wherein a first overlapping portion (F1) is provided between the inner part (9) of the needle cap remover (7) and the outer part (8) of the needle cap remover (7).

7. Drug delivery device (2) according to any of the preceding claims, wherein the outer part (8) comprises a front end (10) having an opening (11) which at its edge exhibits a projection (12) directed inwards adapted to catch a front projection (13) of the inner part (9).

8. Drug delivery device (2) according to any of the preceding claims, wherein the inner part (9) comprises a back end (14) having a grip member (15) adapted to grip a back end (16) of the needle cap (5.1).

9. Drug delivery device (2) according to any of the preceding claims, wherein the projection (12) of the outer part (8) is formed as at least two opposite arms (12.1, 12.2) which ends are angled.

10. Drug delivery device (2) according to any of the preceding claims, wherein the front projection (13) of the inner part (9) is correspondingly formed to the projection (12) of the outer part (8), in particular is formed as at least two protruding tabs (13.1, 13.2).

11. Drug delivery device (2) according to any of the preceding claims, wherein the grip member (15) of the inner part (9) is formed as a back projection of the inner part (9).

12. Drug delivery device (2) according to any of the preceding claims, wherein the grip member (15) of the inner part (9) is formed as a profiled surface adapted to grip lateral surfaces of the needle cap (5.1) by friction connection.

13. Drug delivery device (2) according to any of the preceding claims, wherein the inner part of the needle cap remover (7) is inserted into an opening (6.2) of the needle shield (6) and is also inserted in this opening (6.2) between the needle cap (5.1) and a spring (17) which engages on one end at the housing (2.1) and on the other end at the needle shield (6).
